# EUROPEAN PATENT SPECIFICATION

(11) **EP 2 068 825 B1**
(45) Date of publication and mention of the grant of the patent: **26.01.2011**
(21) Application number: 07818492.6
(22) Date of filing: 27.09.2007
(51) Int. Cl.: A61K 9/00, A61K 9/06, A61K 31/00

(54) **CONTROLLED RELEASE DELIVERY SYSTEM FOR NASAL APPLICATION OF NEUROTRANSMITTERS**
ABGABESYSTEM MIT GESTEUERTER FREISETZUNG ZUR NASALEN ANWENDUNG VON NEUROTRANSMITTERN
SYSTÈME D'ADMINISTRATION À LIBÉRATION CONTRÔLÉE POUR APPLICATION NASALE DE NEUROTRANSMETTEURS

(30) Priority: 04.10.2006 US 828109 P
(43) Date of publication of application: 17.06.2009
(73) Proprietor: M & P Patent Aktiengesellschaft, 9490 Vaduz (LI)
(72) Inventor: MATTERN, Claudia, 6370 Stans (CH)
(74) Representative: Winkler, Andreas Fritz Ernst
(86) International application number: PCT/EP2007/008409
(87) International publication number: WO 2008/040488

(56) References cited:
- EP-A- 0 160 501
- US-B1- 6 310 089

## Description

### FIELD OF THE INVENTION

The invention generally relates to a formulation for the controlled release of neurotransmitters to the systemic circulation and/or to the brain after nasal application. More specifically, the invention relates to the delivery of dopamine to the brain by nasal administration.

### BACKGROUND

A growing body of evidence suggests a modulatory role of brain-acting compounds, such as neurosteroids (e.g., androgens, progestins) or neurotransmitters (e.g., dopamine, 3,4-dihydroxyphenylalanine (L-DOPA)), in the regulation of disorders influenced by receptors in the brain, such as depression, Parkinson's disease, Alzheimer's, or even loss of libido.

Neurosteroids act as modulators of several neurotransmitters, either as stimulators or inhibitors. Neurotransmitters are chemicals that relay, amplify and modulate electrical signals between a neuron and another cell. Some neurotransmitters are excitatory, others primarily inhibitory. In many cases, as with dopamine, it is the receptor which determines whether the transmitter is excitatory or inhibitory.

The brain floats in about 150 ml of CerebroSpinal Fluid (CSF) which slowly circulates down through the four ventricles, up through the subarachinoid space and exits into the cerebral veins through the arachnoid vili. Since the brain has no lymphatic system the CSF serves as a partial substitute. While brain and CSF are separated by the somewhat permeable pia mater, the blood-cerebrospinal fluid barrier and the blood-brain barrier (BBB) represent substantial protection against undesirable blood substances.

The BBB creates a protected chemical environment for the brain wherein certain molecules can perform functions independent of the functions those molecules perform in the rest of the body. This is particularly important e.g. for the neurotransmitter dopamine which, applied as an infusion, is indicated in heart attacks or kidney failure but is not suitable for the treatment of Parkinson's disease since, given by this route (or orally), it is not able to cross the BBB.

To develop effective medicines for disorders of the central nervous system (CNS) nasal delivery has been explored. Whether, after nasal application, an uptake of molecules is via the blood-brain barrier or via direct transport between the nasal cavity and the CSF, or via both is still under debate. In the first case the molecule must traverse the BBB from the systemic circulation after absorption from the nasal mucosa. Since there is also an efflux of molecules from the brain the molecule might appear in the brain and in the blood at different times, with different kinetics or metabolized in different ways.

Nasal drug delivery offers many advantages that include possible direct access to the brain, rapid adsorption due to the abundant presence of capillary vessels in the nose, fast onset of action, avoidance of hepatic first-pass metabolism, utility for chronic medication, and ease of administration. It is also known that, in contrast to large and/or ionized molecules, lipophilic pharmaceutical compounds having a sufficiently low molecular weight generally are readily absorbed by the mucous membrane of the nose.

Since diffusion of drugs into the brain seems to mainly be dependent on their physicochemical properties, for most drugs studied to date the overall amount detected in brain tissue is usually only 2-3% of the nasally administered dose. Thus there has been a long-felt need for investigation of the influence of formulations on this uptake in order to identify formulations that can increase brain availability

However, maintaining constant *in vivo* therapeutic drug concentrations, especially in the brain, for an extended period of time has been problematic. The rapid mucociliary clearance of a therapeutic agent from the site of deposition and the presence of enzymes in the nasal cavity (that may cause degradation of the therapeutic agent) result in a short time span available for absorption. In addition the mechanisms and prerequisites governing direct delivery of molecules to the brain are only poorly understood.

Many efforts have been made in the art in attempt to overcome these limitations. GB 1987000012176 describes the use of bioadhesive microspheres to increase residence time in the nasal cavity. It has also been found that the use of enhancers improves permeability of the nasal membrane and stabilizers prevent drug degradation. PCT/GB98/01147 (U.S. Patent No. 6,432,440) describes the use of *in situ* gelling pectin formulations.

Bayne, U.S. Reissue Patent No. RE29,892 discloses a method of increasing the dopamine concentration in brain tissue through administration of a composition comprising dopamine and a hydrazine compound. The disclosed method allows for administration of the composition topically, rectally, orally, or parenterally. Preferred compositions include hydrazine compounds such as L-α-hydrazino-α-lower alkyl-3, 4-dihydroxyphenyl propionic acid and L-dopa and its pharmacentically accepted salts.

Haffner et al., U.S. Patent No. 4,826,852 discloses methods of administration ergolinyl compounds to increase Dopamine concentrations in mammalian brain tissue. The specific concern of Haffner is the treatment of psychoses such as schizophrenia.

Further, Wenzel et al., U.S. Patent No. 5,624,960, discloses the treatment of Parkinson's disease through the oral administration of a composition containing levodopa and carbidopa. Mandel et al., U.S. Patent No. 6,319,905, discloses the tightly modulated production of L-DOPA in the mammalian brain by gene therapy. Modulators such as tetrahydropterin (PH₄) are used to control the generation of dopamine.

EP 0 160 501 A2 discloses a sustained-release intranasal formulation comprising a catecholamine, an emulsifying agent, a dispersion medium, and a sustained-release agent.

US 6,310,089 B1 discloses a composition for intranasal administration of a partial or full D1-agonist of the dopamine receptor wherein, in an intermediate step for preparation of microparticles, an oil-in-water emulsion is used.

There is a need to identify a formulation that increases the brain's availability to neurotransmitters, particularly dopamine. Neurotransmitters can modulate or control the regulation of emotionality and associated psychiatric disorders, such as depression. The identification of a formulation that could increase the bioavailability of dopamine would open possibilities for the treatment of diseases associated with a deficiency in dopamine in the brain, such as depression, Parkinson's disease, attention deficit hyperactivity disorder (ADHD), or addiction to drugs or alcohol, among others.

### SUMMARY OF THE INVENTION

The inventor has surprisingly found that the incorporation of various neurotransmitter agents into a special lipophilic or partly lipophilic system leads to a higher bioavailability in general caused by sustained serum levels in plasma and CSF.

The invention comprises a formulation for nasal application according to claim 1.

In one aspect of the invention, the active ingredient is a neurotransmitter agent. Preferably, the neurotransmitter is dopamine. It is preferred that dopamine is comprised within the formulation in an amount of from 0.2 to 6% by weight, preferably 0.2 to 4% by weight, more preferably 0.2 to 2% by weight, and most preferably at around 2% by weight.

Preferably, the lipophilic carrier comprises oil. More preferably, the oil is a vegetable oil. Most preferably, the oil is castor oil.

A preferred embodiment of the invention includes oil in an amount of between 30% and 98% by weight, preferably between 60 and 98% by weight, more preferably between 75% and 95% by weight, even more preferably between 85% and 95% by weight and most preferably around 90% by weight of the formulation.

A further embodiment is **characterized in that** the at least one surfactant is selected from the group consisting of lecithin, fatty acid ester of polyvalent alcohols, of sorbitanes, of polyoxyethylensorbitans, of polyoxyethylene, of sucrose, of polyglycerol and/or at least one humectant selected from the group consisting of sorbitol, glycerine, polyethylene glycol, and macrogol glycerol fatty acid ester, or a mixture thereof. Preferably, component (c) comprises an oleoyl macrogolglyceride or a mixture of oleoyl macrogolglycerides.

Component (c) is comprised within the formulation in an amount of from 1 to 20% by weight, preferably 1 to 10% by weight, more preferably 1 to 5% by weight, and most preferably at around 4% by weight.

The formulation of the present invention also comprises a viscosity regulating agent. Preferably, the viscosity regulating agent comprises a thickener or gelling agent selected from the group consisting of cellulose and cellulose derivatives, polysaccharides, carbomers, polyvinyl alcohol, povidone, colloidal silicon dioxide, cetyl alcohols, stearic acid, beeswax, petrolatum, triglycerides and lanolin, or a mixture thereof. More preferably, the viscosity increasing agent is colloidal silicon dioxide.

The viscosity regulating agent is comprised within the formulation in an amount of from 0.5 to 10% by weight, preferably 0.5 to 5% by weight, more preferably 1 to 3% by weight, and most preferably at around 3% by weight.

While not wishing to be bound by theory, it is believed that nasal administration of the galenical formulation of the invention may be able to recruit selective actions of a molecule which, in turn, may provide new clinical applications. It is believed that this effect is due to the access to the brain that is made available by the galenical gel formulation of the invention. Application of the galenical gel formulation of the invention to the nose results in surprising and different action of compounds to the brain as compared to what is seen with conventional formulations.

### BRIEF DESCRIPTION OF THE DRAWINGS

FIG. 1 shows the effect of nasal dopamine gel on immobility and climbing of depressed rats.

FIG. 2 shows the concentration of dopamine and metabolites in nucleus accumbens and neostriatum after nasal administration of dopamine.

### DETAILED DESCRIPTION OF THE INVENTION

The galenical gel formulation of the invention is chemically and physically stable and can be in the form of a suspension or a solution of the pharmacologically active substance. Preferably, the galenical gel formulation of the invention is filled into a preservative-free device able to accurately deliver doses of the above formulation, even at higher viscosities.

After nasal application of the galenical gel formulation of the invention, the active ingredient or active ingredient particles are efficiently trapped at the deposition site and are absorbed at a predictable rate across the mucous membrane of the patient, thereby limiting possible deactivation by metabolizing enzymes and/or protein-binding.

It will also be understood that the terms and expressions used herein have the ordinary meaning as is accorded to such terms and expressions with respect to their corresponding respective areas of inquiry and study except where specific meanings have otherwise been set forth herein.

The term "higher availability" shall mean that after application of a neurotransmitter significant and constant *in vivo* therapeutic drug concentrations , especially in the brain, are maintained for an extended period of time.

### A. Neurotransmitters

The composition of the invention comprises a neurotransmitter. By definition, a neurotransmitter causes rapid, short-lived and dramatic response. Neurotransmitters tend to degrade rapidly, resulting in restoration of the resting membrance potential. Common to many neurotransmitters is a catechol moiety.

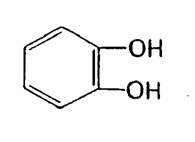

Neurotransmitters such as the epinephrines and dopamine contain the catechol moiety typically synthesized from the amino acid tryosine.

One neurotransmitter commonly associated with any number of responses is dopamine or 4-(2-aminoethyl)-1, 2-benzenediol. Dopamine is commonly regarded as an endogenous catecholamine with a an β andrenergic activity.

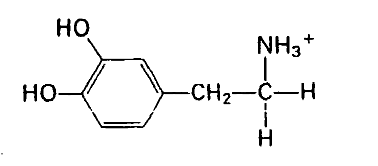

Dopamine or L-DOPA is commonly synthesized from aminotyramine. Other neurotransmitters are generally defined by synaptic vesicles in terminals of the presynaptic neuron and contain the agent, releasing it at the appropriate time in response to stimulation in sufficient quantity to induce response in the post synaptic cell; iontophoresis of the agent into a synaptic cleft induces the same response; and the substance degrades rapidly resulting of restoration of potential.

Other catecholamines known to have various utilities include epinephrine, noreprinephrine, serotonin.

The neurotransmitter drug is comprised within the formulation in an amount of from 0.2 to 6% by weight, preferably 0.2 to 4% by weight, more preferably 0.2 to 2% by weight, and most preferably at around 2% by weight.

### B. Lipophilic Carrier

The carrier of the invention functions to emulsify and otherwise support the various constituents of the invention. To that end, the carrier has a lipophilic nature or character. Suitable families of oils include fatty acids and oils such as mineral and vegetable oils. In particular, fatty acids and oils derived from vegetable stock are especially useful. Both linear and branched chain acids and oils are useful including those oils and acids with various levels of saturation and substitution. Chain size including formic, acetic, propionic, butyric, valeric, caproic, emanthic, caprylic, pelargonic, capric, laurie, myristic, palmitic and stearic, among others, all represent useful oil and acid moieties.

The term "lipophilic carrier" shall comprise, but is not limited to, vegetable oil such as castor oil, soybean oil, sesame oil, or peanut oil, fatty acid esters such as ethyl-and oleyloleat, isopropylmyristate, medium chain triglycerides, glycerol esters of fatty acids, or polyethylene glycol, phospholipids, white soft paraffin, or hydrogenated castor oil, or a mixture thereof. In one aspect, the active ingredient may also be incorporated into an oil mixture. In a preferably aspect, the vegetable oil is castor oil.

The particular amount of lipophilic carrier that constitutes an effective amount is dependent on the particular viscosity regulating agent used in the formulation. It is therefore not practical to enumerate specific amounts for use with specific formulations of the invention. Generally, however, the lipophilic carrier is present in the formulation in an amount between 30% and 98% by weight, preferably between 60 and 98% by weight, more preferably between 75% and 95% by weight, even more preferably between 85% and 95% by weight, and most preferably around 90% by weight of the formulation.

### C. Surface Active Agent

The surface active agent or surfactant of the invention functions to decrease surface tension in the composition of the invention. Surfactants are generally regarded as those compositions which have both hydrophilic and lypophilic character. The lypophilic character of the surfactant tends to take the form of a pendent moriety having little if any charge. In turn, the hydrophilic character of the surfactant tends to be charged and also dictates the class into which the surfactant is identified into. For example, surfactants useful in the invention include nonionic surfactants, anionic surfactants, amphoteric surfactants, and cationic surfactants.

Component (c), a compound or a mixture of compounds having surface tension decreasing activity, comprises at least a surfactant such as, but not limited to, lecithin, fatty acid ester of polyvalent alcohols, fatty acid ester of sorbitanes, fatty acid ester of polyoxyethylensorbitans, fatty acid ester of polyoxyethylene, fatty acid ester of sucrose, fatty acid ester of polyglycerol, and/or at least one humectant such as sorbitol, glycerine, polyethylene glycol, or macrogol glycerol fatty acid ester. Particularly useful, however, are oleoyl macrogolglycerides (such as LABRAFIL^{®} M 1944 CS, as available from Gattefossé (Saint-Priest, France)).

In another aspect, the active ingredient may be incorporated into a surfactant mixture. The particular amount of surfactant that constitutes an effective amount is dependent on the particular oil or oil mixture used in the formulation. It is therefore not practical to enumerate specific amounts for use with specific formulations of the invention. Generally, however, the surfactant is present in a formulation in an amount of from 1 to 20% by weight, preferably 1 to 10% by weight, more preferably 1 to 5% by weight, and most preferably at around 4% by weight.

### D. Viscosity Regulating Agent

The term "viscosity regulating agent" shall mean a thickener or gelling agent. Examples are, but not limited to, cellulose and derivatives thereof, polysaccharides, carbomers, polyvinyl alcohol, povidone, colloidal silicon dioxide, cetyl alcohols, stearic acid, beeswax, petrolatum, triglycerides, lanolin, or the like. A preferred viscosity regulating agent is colloidal silicon dioxide (such as ACROSIL 200^{®}, as available from Degussa).

The incorporation of the active ingredient is also possible into a mixture of thickeners or gelling agents. The particular amount of thickener/gelling agent that constitutes an effective amount is dependent on the particular oil or oil mixture used in the formulation. It is therefore not practical to enumerate specific amounts for use with specific formulations of the invention. Generally, however, the thickener/gelling agent(s) is present in a formulation in an amount from 0.5 to 10% by weight, preferably 0.5 to 5% by weight, more preferably 1 to 3% by weight, and most preferably at around 3% by weight.

**TABLE**

| Wt-% | | | |
|---|---|---|---|
| | Useful | Preferred | More Preferred |
| Neurotransmitter | 0.5 to 6 | 2 to 4 | 0.5 to 2 |
| Lipophilic Carrier | 30 to 98 | 60 to 98 | 85 to 95 |
| Surfactant | 1 to 20 | 1 to 10 | 1 to 5 |
| Viscosity Regulating Agent | 0.5 to 10 | 0.5 to 5 | 1 to 3 |

### Formulation

Generally, the galenical formulation of the invention can be prepared very easily by the following conventional method:

The lipophilic carrier and emulsifier are filled into a stirrer vessel and about 75% of the viscosity regulating agent is mixed in. The active ingredient is added under stirring to obtain a homogenous dispersion of the active ingredient. Next, the formulation is adjusted to the necessary viscosity with the remainder of the viscosity regulating agent.

The formulation is preferably filled into a preservative-free container.

Because neurotransmitters may have lower levels of solubility in water, liberation from the formulation is the speed-limiting step for adsorption. It has been surprisingly found that the incorporation of the active agent in an oily formulation containing a suitable surfactant according to the invention leads to physiologic serum levels and to a steady, sustained action of the hormone over time.

The release of the neurotransmitter agent is sustained due to its solubility in the oily carrier and because the formulation remains on the mucous membrane for a prolonged duration of time due to its viscosity.

Upon contact of the formulation with the humidity of the mucous membrane, precipitation of the active ingredient is hindered by the surfactant's ability to form oil drops containing the active ingredient. Thus, by adding a suitable surfactant to the formulation, the dissolution pattern of the active ingredient becomes more favorable and effective because there is no big variability in dissolution, which ensures bioequivalence.

The active ingredient of this invention may be introduced into the formulation also in a processed form, such as microspheres, liposomes, among others.

The formulation according to this invention may also be processed into powder form, such as by lyophilization or spray-drying.

**Table 1. Most Preferred formulation**

| Compound | Amount per container | Delivery per spray |
|---|---|---|
| Dopamine | 2% | ≈ 2.8 mg |
| Colloidal silicon dioxide | 3% | ≈ 4.2 mg |
| Oleoyl macrogol-glycerides | 4% | ≈ 5.6 mg |
| Castor oil | 91% | ≈ 127.4 mg |

### EXAMPLES

The following examples are intended to further illustrate, and not limit, embodiments in accordance with the invention.

### Example 1: Nasal administration of dopamine to rats

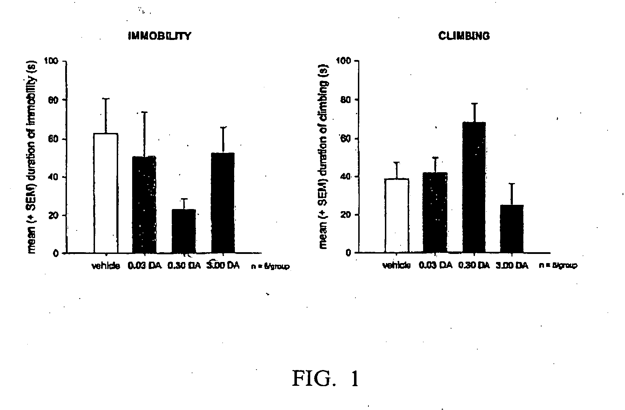

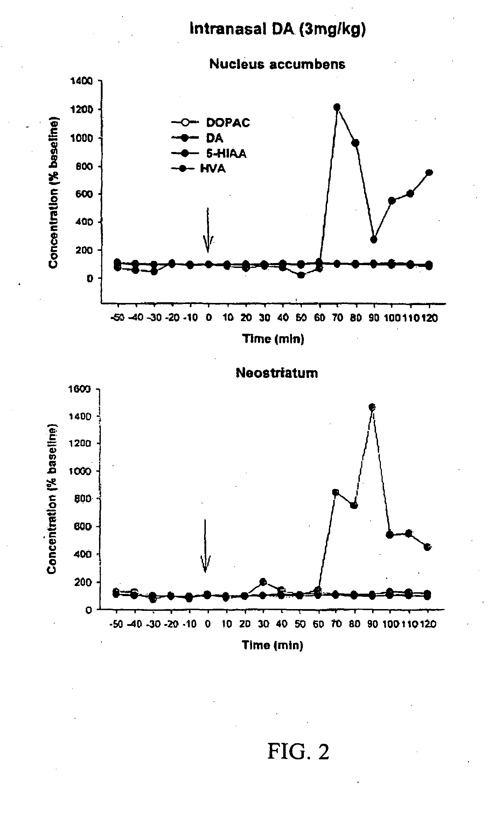

A dopamine (DA) gel of the inventive formulation was nasally administered to rats used in the validated "forced swimming test." As shown in FIG.1, the administration of dopamine results in anti-depressive-like effects. As shown in FIG. 2, strong dopaminergic activity in the neostriatum and ventral striatum (nucleus accumbens) is seen after nasal application of dopamine with the inventive formulation.

Generally, antidepressants must be taken for longer time before antidepressive effects are seen. Surprisingly, after nasal application of the dopamine gel formulation to rats, antidepressive effects occurred within hours and without any side effects, such as those side effects known to occur with desipramine (apathy) or fluoxetine (weight loss).

After nasal application of dopamine in the inventive gel formulation of the invention to rats, it was surprisingly found that the concentration of dopamine in the nucleus accumbens and neostriatum level increases very rapidly by more than 1000 percent. These results are different from those previously described. After nasal application of an aqueous dopamine solution to mice, Bjorn Jansson, Comprehensive Summaries of Uppsala Dissertations from the Faculty of Pharmacy 305 (2004), found dopamine in the olfactory bulb but the compound peaked after four hours. After nasal application of aqueous dopamine solution to rats, Maria Dahlin, Comprehensive Summaries of Uppsala Dissertations from the Faculty of Pharmacy 240 (2000), found dopamine in cerebrospinal fluid (CSF) after a short time but the increase of the compound from baseline is much lower than with the nasal gel of the invention. Ikeda et al., Chem. Pharm. Bull. 40(8): 2155-2158 (1992), increased bioavailability of nasally-given dopamine by some degree using the excipients hydroxypropyl cellulose (HPC) and Azone (1-dodecylazacycloheptan-2-one), respectively. De Souza Silva et al., Synapse 27:294-302 (1997), showed that by nasally applying an aqueous L-DOPA methyl ester solution to rats (50 mg/kg), the dopamine level in the neostriatum could be increased by about 130%. The metabolites 3,4-dihydroxyphenyl acetic acid (DOPAC) and homovanillic acid (HVA) slightly increased in contrast to what was seen after ip application of L-DOPA methyl ester in De Souza Silva et al., J. Neurochem. 68(1): 233-239 (1997).

Furthermore, the metabolism of dopamine appears to be quite different than that previously described. Very unexpectedly, as shown in FIG. 2, dopamine in the cerebral spinal fluid (CSF) is not metabolized to 3, 4-dihydroxyphenyl acetic acid (DOPAC) or homovanillic acid (HVA) as is usually seen. These results demonstrate that nasal application of dopamine in the inventive gel formulation may thus be useful for the causal treatment of diseases associated with dopamine deficiency in the brain, such as Parkinson's disease, attention deficit hyperactivity disorder (ADHD), or addition to drugs and/or alcohol.

## Claims

1. A formulation for nasal application comprising a) at least one neurotransmitter; b) at least one lipophilic carrier; and c) at least one surfactant, in an amount effective for in situ generation of an emulsion upon contact of the formulation with water, the formulation further comprising a viscosity regulating agent.

2. The formulation according to claim 1, wherein the lipophilic carrier comprises an oil.

3. The formulation according to claim 2, wherein the oil is a vegetable oil.

4. The formulation according to claim 3, wherein the vegetable oil is castor oil.

5. The formulation according to claim 1, wherein the amount of component (b) comprises between 30% and 98% by weight of the formulation, preferably between 60 and 98% by weight, more preferably between 75% and 95% by weight, even more preferably between 85% and 95% by weight and most preferably around 90% by weight of the formulation.

6. The formulation according to claim 1, wherein the at least one surfactant is selected from the group consisting of lecithin, fatty acid ester of polyvalent alcohols, of sorbitanes, of polyoxyethylensorbitans, of polyoxyethylene, of sucrose, of polyglycerol and/or at least one humectant selected from the group consisting of sorbitol, glycerine, polyethylene glycol, and macrogol glycerol fatty acid ester, or a mixture thereof.

7. The formulation according to claim 6, wherein component (c) comprises an oleoyl macrogolglyceride or a mixture of oleoyl macrogolglycerides.

8. The formulation according to claim 1, wherein component (c) is comprised within the formulation in an amount of from 1 to 20% by weight, preferably 1 to 10% by weight, more preferably I to 5% by weight, and most preferably at around 4% by weight.

9. The formulation according to claim 1, wherein said viscosity regulating agent comprises a thickener or gelling agent selected from the group consisting of cellulose and cellulose derivatives, polysaccharides, carbomers, polyvinyl alcohol, povidone, colloidal silicon dioxide, cetyl alcohols, stearic acid, beeswax, petrolatum, triglycerides and lanolin, or a mixture thereof.

10. The formulation according to claim 9, wherein said viscosity regulating agent is colloidal silicon dioxide.

11. The formulation according to claim 1, wherein the viscosity regulating agent is comprised within the formulation in an amount of from 0.5 to 10% by weight, preferably 0.5 to 5% by weight, more preferably 1 to 3% by weight, and most preferably at around 3% by weight.

12. The formulation according to claim 1, wherein the neurotransmitter is dopamine.

13. The formulation according to claim 1, wherein the neurotransmitter is comprised within the formulation in an amount of from 0.2 to 6% by weight, preferably 0.2 to 4% by weight, more preferably 0.2 to 2% by weight, and most preferably at around 2% by weight.

## Patentansprüche

1. Formulierung zur nasalen Anwendung, umfassend a) wenigstens einen Neurotransmitter; b) wenigstens einen lipophilen Trägerstoff; und c) wenigstens ein oberflächenaktives Mittel, in einer Menge, die zur in-situ-Erzeugung einer Emulsion bei Kontakt der Formulierung mit Wasser wirksam ist, wobei die Formulierung weiter ein die Viskosität regulierendes Mittel umfasst.

2. Formulierung nach Anspruch 1, wobei der lipophile Trägerstoff ein Öl umfasst.

3. Formulierung nach Anspruch 2, wobei das Öl ein pflanzliches Öl ist.

4. Formulierung nach Anspruch 3, wobei das pflanzliche Öl Rizinusöl ist.

5. Formulierung nach Anspruch 1, wobei die Menge von Komponente (b) zwischen 30 und 98 Gew.-% der Formulierung, vorzugsweise zwischen 60 und 98 Gew.-%, bevorzugter zwischen 75 und 95 Gew.-%, noch bevorzugter zwischen 85 und 95 Gew.-% und am bevorzugtesten etwa 90 Gew.-% der Formulierung umfasst.

6. Formulierung nach Anspruch 1, wobei das wenigstens eine oberflächenaktives Mittel ausgewählt ist aus der Gruppe, bestehend aus Lecithin, Fettsäureester von mehrwertigen Alkoholen, von Sorbitanen, von Polyoxyethylensorbitanen, von Polyoxyethylen, von Saccharose, von Polyglycerol und/oder wenigstens einem Feuchthaltemittel, das ausgewählt ist aus der Gruppe, bestehend aus Sorbitol, Glycerin, Polyethylenglykol und Macrogolglycerolfettsäureester, oder einer Mischung davon.

7. Formulierung nach Anspruch 6, wobei Komponente (c) ein Oleoylmacrogolglycerid oder eine Mischung von Oleoylmacrogolglyceriden umfasst.

8. Formulierung nach Anspruch 1, wobei Komponente (c) in der Formulierung in einer Menge von 1 bis 20 Gew.-%, vorzugsweise 1 bis 10 Gew.-%, bevorzugter 1 bis 5 Gew.-% und am bevorzugtesten mit etwa 4 Gew.-% enthalten ist.

9. Formulierung nach Anspruch 1, wobei das die Viskosität regulierende Mittel ein Verdickungsmittel oder Geliermittel umfasst, das ausgewählt ist aus der Gruppe, bestehend aus Cellulose und Cellulosederivaten, Polysacchariden, Carbomeren, Polyvinylalkohol, Povidon, kolloidalem Siliciumdioxid, Cetylalkoholen, Stearinsäure, Bienenwachs, Petrolatum, Triglyceriden und Lanolin, oder eine Mischung davon.

10. Formulierung nach Anspruch 9, wobei das die Viskosität regulierende Mittel kolloidales Siliciumdioxid ist.

11. Formulierung nach Anspruch 1, wobei das die Viskosität regulierende Mittel in der Formulierung in einer Menge von 0,5 bis 10 Gew.-%, vorzugsweise 0,5 bis 5 Gew.-%, bevorzugter 1 bis 3 Gew.-% und am bevorzugtesten mit etwa 3 Gew.-% enthalten ist.

12. Formulierung nach Anspruch 1, wobei der Neurotransmitter Dopamin ist.

13. Formulierung nach Anspruch 1, wobei der Neurotransmitter in der Formulierung in einer Menge von 0,2 bis 6 Gew.%, vorzugsweise 0,2 bis 4 Gew.-%, bevorzugter 0,2 bis 2 Gew.-% und am bevorzugtesten mit etwa 2 Gew.-% enthalten ist.

## Revendications

1. Formulation pour application nasale comprenant a) au moins un neurotransmetteur ; b) au moins un véhicule lipophile ; et c) au moins un tensioactif, en une quantité efficace pour la génération in situ d'une émulsion lors du contact de la formulation avec de l'eau, la formulation comprenant, en outre, un agent de régulation de la viscosité.

2. Formulation selon la revendication 1, dans laquelle le véhicule lipophile comprend une huile.

3. Formulation selon la revendication 2, dans laquelle l'huile est une huile végétale.

4. Formulation selon la revendication 3, dans laquelle l'huile végétale est l'huile de ricin.

5. Formulation selon la revendication 1, dans laquelle la quantité de composant (b) représente entre 30 et 98 % en poids de la formulation, de préférence, entre 60 et 98 % en poids, plus préférablement, entre 75 et 95 % en poids, plus préférablement encore, entre 85 et 95 % en poids, et de manière préférée entre toutes, environ 90 % en poids de la formulation.

6. Formulation selon la revendication 1, dans laquelle ledit au moins tensioactif est choisi dans le groupe constitué par la lécithine, l'ester d'acide gras des polyols, des sorbitans, des polyoxyéthylène-sorbitans, du polyoxyéthylène, du saccharose, du polyglycérol et/ou au moins un humidifiant choisi dans le groupe constitué par le sorbitol, le glycérol, le polyéthylène glycol et l'ester d'acide gras du macrogol glycérol ou leur mélange.

7. Formulation selon la revendication 6, dans laquelle le composant (c) comprend un macrogolglycéride d'oléoyle ou un mélange de macrogolglycérides d'oléoyle.

8. Formulation selon la revendication 1, dans laquelle le composant (c) est compris dans la formulation en une quantité de 1 à 20 % en poids, de préférence, de 1 à 10 % en poids, plus préférablement, de 1 à 5 % en poids, et de manière préférée entre toutes, d'environ 4 % en poids.

9. Formulation selon la revendication 1, dans laquelle ledit agent de régulation de la viscosité comprend un épaississant ou un gélifiant choisi dans le groupe constitué par la cellulose et les dérivés de cellulose, les polysaccharides, les carbomères, l'alcool polyvinylique, la povidone, le dioxyde de silicium colloïdal, les alcools cétyliques, l'acide stéarique, la cire d'abeilles, la vaseline, les triglycérides et la lanoline ou leur mélange.

10. Formulation selon la revendication 9, dans laquelle ledit agent de régulation de la viscosité est le dioxyde de silicium colloïdal.

11. Formulation selon la revendication 1, dans laquelle l'agent de régulation de la viscosité est compris dans la formulation en une quantité de 0,5 à 10 % en poids, de préférence, de 0,5 à 5 % en poids, plus préférablement, de 1 à 3 % en poids, et de manière préférée entre toutes, d'environ 3 % en poids.

12. Formulation selon la revendication 1, dans laquelle le neurotransmetteur est la dopamine.

13. Formulation selon la revendication 1, dans laquelle le neurotransmetteur est compris dans la formulation en une quantité de 0,2 à 6 % en poids, de préférence, de 0,2 à 4 % en poids, plus préférablement, de 0,2 à 2 % en poids, et de manière préférée entre toutes, d'environ 2 % en poids.
